# EUROPEAN PATENT SPECIFICATION

(11) **EP 1 896 130 B1**
(45) Date of publication and mention of the grant of the patent: **24.11.2010**
(21) Application number: 05769378.0
(22) Date of filing: 17.06.2005
(51) Int. Cl.: A61N 5/06

(54) **A DEVICE FOR HUMAN BODY TREATMENT BY ELECTROMAGNETIC WAVES**
VORRICHTUNG ZUR BEHANDLUNG DES MENSCHLICHEN KÖRPERS MIT ELEKTROMAGNETISCHEN WELLEN
DISPOSITIF POUR LE TRAITEMENT DU CORPS HUMAIN AVEC DES ONDES ÉLECTROMAGNÉTIQUES

(43) Date of publication of application: 12.03.2008
(73) Proprietor: Baldacchini, Marcello Rinaldo, 25135 S. Eufemia (BS) (IT)
(72) Inventor: Baldacchini, Marcello Rinaldo, 25135 S. Eufemia (BS) (IT)
(74) Representative: Pulieri, Gianluca Antonio
(86) International application number: PCT/IT2005/000350
(87) International publication number: WO 2006/134620

(56) References cited:
- CH-A- 685 148
- US-A- 5 231 984
- US-A- 5 616 140
- US-A- 5 698 866
- US-B1- 6 471 716

## Description

. The present invention relates to a device for human body treatment by electromagnetic waves.

. Several solutions are known relating to laboratory devices for human body treatment by waves in the nIR (near Infra-red) or IR (Infra-red) range, generated by laser. Such devices are generally very bulky, expensive, requiring skilled personnel, and therefore are not suitable for home use, for example every day.

. There are also solutions reflating to devices suitable for home use, generally comprising a brush to move on the part of the body to be treated.

. Solutions of the type described above are for example described in documents WO97/09847, RU2231353, DE4012854, WO2004/075985, WO2005/016454, CN1369239, GB2368020, US5,336,159, EP1005880, DE4143168, US4,680,822, RU2232610, DE4113803, GB2212010, WO02/053224, TW580916 and CH685148A.

. However, known solutions exhibit several disadvantages.

. The object of the present invention is to overcome the disadvantages of prior art devices and to provide a device for the therapeutic treatment of human or animal body, particularly suitable for a home use.

. Such object is achieved by a device obtained according to claim 1. The dependent claims describe embodiment variations. Moreover, said device can be used for carrying out a method for treating the human body.

. The features and advantages of the device according to the present invention will appear more clearly from the following description, made by way of an indicative non-limiting example with reference to the following figures, wherein:

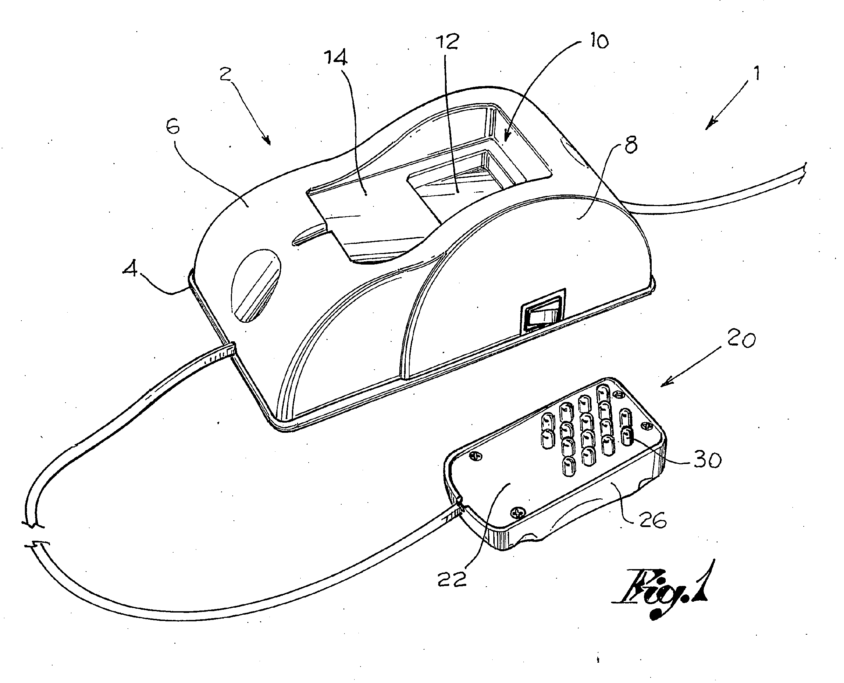

. - figure 1 shows a perspective view of the device according to the present invention;

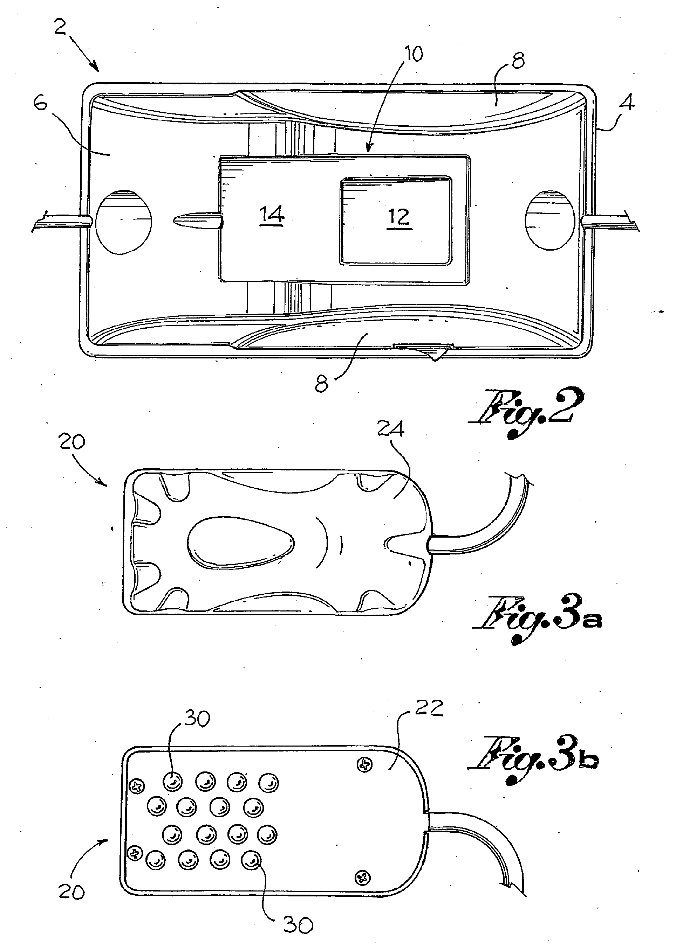

. - figure 2 shows a plan view of a seating body of device of figure 1, and

. - figures 3a and 3b show plan views of a handling case of device of figure 1.

. With reference to the annexed figures, reference numeral 1 globally indicates a device for human body treatment by electromagnetic waves, said device being suitable for being used on a part of said body to be treated.

. Device 1 comprises a seating body 2 suitable for resting on a holder.
According to a preferred embodiment, said body 2 is a box structure infernally provided with a space. Said box structure comprises a support base 4, suitable for resting on said holder, an upper wall 6, opposed to said support base, and side walls 8. Said support base 4, said upper wall 6 and said side walls 8 delimit the inside space.

. According to a preferred embodiment, said upper wall 6 of body 2 exhibits a seat 10, wherein a lower space 12 and an upper space 14 can be distinguished.

. According to a preferred embodiment, said device 1 comprises power supply means. For example, said power supply means are suitable for being connected to the mains for powering said device. In accordance with an embodiment variation, said power supply means comprise at least one battery, for example of the "rechargeable" type, for powering device 1.

. Moreover, device 1 comprises a handling case 20, operatively connected to said seating body 2 and suitable for being held in a hand for being handled and for carrying out said treatment.

. According to a preferred embodiment, said handling case 20 exhibits a front surface 22, intended for facing the part to be treated during a treatment, a back surface 24, opposed to said front surface, and side surfaces 26.

. Moreover, said device 1 comprises substantially monochromatic emission means suitable for emitting a substantially monochromatic wave.

. In particular, said emission means comprise a plurality of separate emitters 30, each emitter being suitable for emitting a wave having a predetermined wavelength.

. Preferably, said emitters are suitable for emitting an electromagnetic wave having a wavelength equal to the characteristic wavelength of silicon emission, that is, substantially equal to 905 nanometres.

. Within the scope of this invention, the term "substantially equal" indicates that, for intrinsic technological constraints, the value of the wavelength emitted by an emitter may differ from the indicated value, but will in any case remain around said value. In particular, according to an embodiment, said wavelength is comprised in a range between 875 nanometres and 935 nanometres.

. Preferably, said emitters are light emitting diodes (LED).

. Emitters 30 are arranged on said case 20, for impinging at least partly, with said waves, the part of the body to be treated, during the treatment.

. Preferably, said emitters 30 are projecting from said front surface 22 of case 20 and spaced from each other.

. In particular, emitters 30 are arranged so as to generate a matrix wherein rows and columns can be identified. Emitters 30 of a row are arranged in an intermediate position relative to the emitters of an adjacent row, with adjacent row or adjacent column to a previous row/column meaning the row/column directly next to the previous row/column.

. In particular, an emitter 30 not arranged on a peripheral row or on a peripheral column of the matrix, with peripheral row/column meaning the lat row/column of the matrix, that is, a row/column that, at least on one side, has no adjacent row/column, exhibits a constant distance relative to adjacent emitters.

. In other words, said emitters 30 exhibit a honeycombed arrangement.

. Said seating body 2 is suitable for seating, at least partly, said case 20, for keeping said device in a non-use condition. In particular, said lower space is suitable for seating said emitters 30, whereas said upper space is suitable for seating said case.

. According to a preferred embodiment, said device 1 comprises primary supply means and secondary supply means.

. Said secondary supply means seated into said case 20, are suitable for powering said emission means and in particular, said emitters. Preferably, said secondary supply means are suitable for powering said emitters with a predetermined power supply frequency, preferably matching the mains frequency.

. On the other hand, said primary supply means, operatively connectable to said power supply means, are seated into said body 2 and operatively connected to said secondary supply means.

. In the standard use of the device described above, the device is connected to the mains or powered by battery.

. When the device is switched on, said emitters emit a wave falling in the nIR range, preferably equal to the characteristic silicon wavelength, that is, substantially equal to 905 nanometres.

. The emission of a wave at the above wavelength can be testable using a special detecting device. nIR waves, in fact, are not within the visible spectrum and therefore are not perceived by the human eye. However, as detecting device it is possible to use a digital camera of the type available on the market, whose lens is sensitive to nIR waves.

. Staring at the emitters with naked eye, when the device is operating, it will therefore not be possible to detect any light. On the other hand, framing the emitters with the camera, a light corresponding to the emission of the above wave will be visible.

. For the human body treatment, the case is moved close to the part of the body to be treated, aiming the emitters at the part of the body to be treated.

. Moving the case on the part to be treated, in contact with, lightly touching or a little away from, the skin, the emitted waves impinge the part to be treated, causing a resonant effect in the cells forming the part to be treated.

. The electromagnetic waves generated, having a wavelength substantially equal to the silicon wavelength, can be used for treating alopecia, rheumatic disorders, for dermatological and aesthetic treatments and the like. Such use, moreover, has shown considerable results for obtaining the vasodilatation of the penis capillaries.

. Advantageously, the arrangement of the emitters on the front surface of the case allows obtaining an even distribution of the intensity of the waves impinging the part of the body to be treated. Advantageously, such feature allows not causing any undesired reactions on the part of the body to be treated, for example nerve stressing due to an excessive difference of electrical potential generated between adjacent portions of the part of the body to be treated.

. Advantageously, moreover, when not temporarily used, for example during a treatment, the device is suitable for being arranged in a non-use configuration wherein the emitters are seated in the lower space of the seat obtained in the seating body. In this way, the emitted waves are confined into said seat.

. Advantageously, moreover, it has been found that the use of the described device on a part of the body where a cream or oil or in general, a substance reacting with said part of the body has been applied before and/or after, for the therapeutic or aesthetic treatment thereof, considerably stimulates the reaction of said substance with said part of the body.

. The device according to the present invention is preferably marketed as a part of an assembly comprising said device and at least one protective cover.

. According to a preferred embodiment, said protective cover comprises a deformable bag suitable for seating, at least partly, said case 20. Preferably, said bag is transparent to the emitted waves and is made of a plastic material, for example polyethylene.

. Before carrying out the treatment, case 20, or the part of said case exhibiting emitters 30, is inserted into said bag and then moved close to the part of the body to be treated.

. At the end of the treatment, the case is removed from the bag, which is disposed.

. Advantageously, the assembly allows using the device for carrying out different treatments on different parts of the body or on different users.

. In particular, the assembly allows using specific creams or products for each part of the body, avoiding soiling the emitters and consequently, polluting other parts of the body treated afterwards. Of course, thins also allows use by different users, protecting hygiene.

. In other words, a portion of the bag provides a wall transparent to the waves, for preventing physical contact between said emitters and said part of the body to be treated.

. Advantageously, moreover, said bag also provides a further electrical and chemical insulation between the device and the part of the body to be treated.

. Finally, according to a further advantageous aspect thereof, said power supply of said emitters at the main frequency enhances the wave emission, decreasing the emission time, allowing a penetration of said waves into the part of the body to be treated.

. It is clear that a man skilled in the art can make several changes and adjustments to the device described above in order to meet specific and incidental needs, all falling within the scope of protection defined in the following claims.

## Claims

1. A device (1) for human or animal body treatment, said device being suitable for being used on a part of said body to be treated and comprising:
- a seating body (2) suitable for resting on a holder, wherein said seating body is suitable for seating, at least partly, a case, for keeping said device in a non-use condition;
- a handling case (20), operatively connected to said seating body (2) and suitable for being held in a hand for being handled and for carrying out said treatment;
- emission means, said emission means comprising a plurality of separate emitters (30), each emitter being suitable for emitting an electromagnetic wave having a wavelength comprised in the range 875 - 935 nanometres, wherein said emitters are arranged on said case (20), for impinging with said waves, during said treatment, said part of the body to be treated,
wherein the emission means are emitting substantially monochromatic electromagnetic waves,
wherein said emitters (30) are arranged so as to generate a matrix wherein rows and columns can be identified,
and wherein the emitters of a row are arranged in intermediate position relative to the emitters of an adjacent row;
**characterized by** the fact that said seating body (2) exhibits a support base (4), suitable for resting on said holder, and an upper wall (6), opposed to said support base (4), wherein said upper wall (6) exhibits a seat (10) for seating said case (20).

2. A device (1) according to claim 1, wherein said emitters (30) are suitable for emitting a wave having a wavelength substantially equal to the characteristic wavelength of silicon emission.

3. A device (1) according to claim 2, wherein said emitters (30) are suitable for emitting a wave having a wavelength equal to 905 nanometres.

4. A device (1) according to any one of the previous claims, wherein said handling case (20) exhibits a front surface (22), intended for facing the part to be treated during a treatment, and a back surface (24), said emitters being arranged on the side of said front surface (22).

5. A device (1) according to claim 4, wherein said emitters (30) are projecting from said front surface (22).

6. A device (1) according to any one of the previous claims, wherein said emitters (30) are spaced from one another.

7. A device (1) according to any one of the previous claims, wherein an emitter (30) not arranged on a peripheral row or on a peripheral column, exhibits a constant distance relative to adjacent emitters (30).

8. A device (1) according to any one of the previous claims, wherein said emitters (30) exhibit a honeycombed arrangement.

9. A device (1) according to any one of the previous claims , wherein said seat (10) exhibits a lower space (12) and an upper space (14), that can be distinguished from each other, said lower space (12) being suitable for seating said emitters (30) and said upper space (14) being suitable for seating, at least partly, said case (20).

10. A device (1) according to any one of the previous claims, comprising secondary supply means suitable for powering said emitters (30) for emitting said wave.

11. A device (1) according to claim 10, wherein said power supply means are seated into said case (20).

12. A device (1) according to any one of the previous claims, comprising primary supply means, suitable for cooperating with said secondary supply means, for powering said secondary supply means.

13. A device (1) according to claim 12, wherein said primary supply means are seated into said seating body (2).

14. A device (1) according to any one of claims from 10 to 13, wherein said secondary supply means are suitable for powering said emitters powering them at a predetermined frequency.

15. A device (1) according to claim 14, wherein said secondary supply means are suitable for powering said emitters powering them at a frequency equal to the mains frequency.

16. A device (1) according to any one of the previous claims, wherein said emitters (30) are light emitting diodes.

17. An assembly for human or animal body treatment, comprising at least one device (1) according to any one of the previous claims and at least one wall transparent to waves, for preventing physical contact between said emitters (30) and said part of the body to be treated.

18. An assembly according to claim 17, wherein said transparent wall is a portion of a bag.

19. An assembly according to claim 17 or 18, wherein said wall is made of polyethylene.

## Patentansprüche

1. Vorrichtung (1) zur Behandlung des menschlichen oder tierischen Körpers, wobei die Vorrichtung zur Verwendung an einem zu behandelnden Abschnitt des Körpers geeignet ist und umfassend
- einen Auflagekörper (2), der zum Ruhen auf einem Halter geeignet ist, wobei der Auflagekörper wenigstens teilweise zum Auflegen eines Gehäuses geeignet ist, um die Vorrichtung in einem nicht gebrauchten Zustand zu halten,
- ein Handhabungsgehäuse (20), das mit dem Auflagekörper (2) wirkungsmäßig verbunden ist und zum Halten in einer Hand geeignet ist, um gehandhabt und zum Ausführen der Behandlung verwendet zu werden,
- Emissionsmittel, die eine Mehrzahl von separaten Emittern (30) umfassen, wobei jeder Emitter zur Ausstrahlung einer elektromagnetischen Welle, die eine Wellenlänge aufweist, die in den Bereich von 875 - 935 Nanometer fällt, geeignet ist,
wobei die Emitter dazu an dem Gehäuse (20) angeordnet sind, um den zu behandelnden Abschnitt des Körpers während der Behandlung mit den Wellen zu beaufschlagen, wobei die Emissionsmittel im Wesentlichen monochormatische, elektromagnetische Wellen aussenden, wobei die Emitter (30) derart angeordnet sind, dass sie eine Matrix bilden, wobei Reihen und Spalten gebildet werden können,
und wobei die Emitter einer Reihe in einer Zwischenposition zu den Emittern einer benachbarten Reihe angeordnet sind,
**dadurch gekennzeichnet, dass** der Auflagekörper (2) eine Stützbasis (4), die zur Auflage auf dem Halter geeignet ist, und eine obere Wand (6), die der Stützbasis (4) gegenüberliegt, aufweist, wobei die obere Wand (6) einen Sitz (10) zur Aufnahme des Gehäuses (20) aufweist.

2. Vorrichtung (1) gemäß Anspruch 1, wobei die Emitter (30) zur Austrahlung einer Welle geeignet sind, die eine Wellenlänge aufweist, die im Wesentlichen gleich der charakteristischen Wellenlänge der Emission von Silikon ist.

3. Vorrichtung (1) gemäß Anspruch 2, wobei die Emitter (30) zur Ausstrahlung einer Welle, die eine Wellenlänge aufweist, die gleich 905 Nanometer ist, geeignet sind.

4. Vorrichtung (1) gemäß irgend einem der vorangehenden Ansprüche, wobei das Handhabungsgehäuse (20) eine Vorderfläche (22), die dem zu behandelnden Abschnitt während einer Behandlung zugewandt sein soll, und eine Rückfläche (24) aufweist, wobei die Emitter an der Seite der Vorderfläche (22) angeordnet sind.

5. Vorrichtung (1) gemäß Anspruch 4, wobei die Emitter (30) von der Vorderfläche (22) vorragen.

6. Vorrichtung (1) gemäß irgend einem der vorangehenden Ansprüche, wobei die Emitter (30) voneinander beabstandet sind.

7. Vorrichtung (1) gemäß irgend einem der vorangehenden Ansprüche, wobei ein Emitter (30), der nicht in einer Umfangsreihe oder einer Umfangsspalte angeordnet ist, einen konstanten Abstand zu den benachbarten Emittern (30) aufweist.

8. Vorrichtung (1) gemäß irgend einem der vorangehenden Ansprüche, wobei die Emitter (30) eine wabenartige Anordnung aufweisen.

9. Vorrichtung (1) gemäß irgend einem der vorangehenden Ansprüche, wobei der Sitz (10) einen unteren Freiraum (12) und einen oberen Freiraum (14) aufweist, die voneinander unterschieden werden können, wobei der untere Freiraum (12) zur Aufnahme der Emitter (30) und der obere Freiraum (14) zur wenigstens teilweisen Aufnahme des Gehäuses (20) geeignet ist.

10. Vorrichtung (1) gemäß irgend einem der vorangehenden Ansprüche umfassend Sekundärversorgungsmittel, die zur Stromversorgung der Emitter (30) zur Ausstrahlung der Welle geeignet sind.

11. Vorrichtung (1) gemäß Anspruch 10, wobei die Stromversorgungsmittel in dem Gehäuse (20) sitzen.

12. Vorrichtung (1) gemäß irgend einem der vorangehenden Ansprüche, umfassend Primärversorgungsmittel, die zum Zusammenwirken mit den Sekundärversorgungsmitteln zur Stromversorgung der Sekundärversorgungsmittel geeignet sind.

13. Vorrichtung (1) gemäß Anspruch 12, wobei die Primärversorgungsmittel in dem Auflagekörper (2) sitzen.

14. Vorrichtung (1) gemäß irgend einem der vorangenden Ansprüche 10 bis 13, wobei die Sekundärversorgungsmittel zur Stromversorgung der Emitter geeignet sind und diese mit Strom mit einer vorbestimmten Frequenz versorgen.

15. Vorrichtung (1) gemäß Anspruch 14, wobei die Sekundärstromversorgungsmittel zur Stromversorgung der Emitter mit einem Strom mit einer vorbestimmten Frequenz, die gleich der Netzfrequenz ist, geeignet sind.

16. Vorrichtung (1) gemäß irgend einem der vorangehenden Ansprüche, wobei die Emitter (30) Leuchtdioden (LEDs) sind.

17. Anordnung zur Behandlung eines menschlichen oder tierischen Körpers, umfassend wenigstens eine Vorrichtung (1) gemäß irgend einem der vorangehenden Ansprüche und wenigstens eine Wand, die für Wellen durchlässig ist, um einen physischen Kontakt zwischen den Emittern (30) und dem zu behandelnden Abschnitt des Körpers zu verhindern.

18. Anordnung gemäß Anspruch 17, wobei die durchlässige Wand Teil eines Beutels ist.

19. Anordnung gemäß Anspruch 17 oder 18, wobei die Wand aus Polyethylen hergestellt ist.

## Revendications

1. Dispositif (1) destiné au traitement d'un corps humain ou animal, ledit dispositif étant approprié de façon à être utilisé sur une partie dudit corps à traiter et comprenant :
- un corps de réception (2) approprié à reposer sur un support, dans lequel ledit corps de réception est approprié de façon à recevoir, au moins en partie, un boîtier, afin de maintenir ledit dispositif dans un état de non utilisation;
- un boîtier de manipulation (20), connecté de manière opérationnelle audit corps de réception (2) et approprié de façon à être tenu dans une main pour être manipulé et pour exécuter ledit traitement;
- des moyens d'émission, lesdits moyens d'émission comprenant une pluralité d'émetteurs séparés (30), chaque émetteur étant approprié de façon à émettre une onde électromagnétique qui présente une longueur d'onde qui se situe dans la plage comprise entre 875 nanomètres et 935 nanomètres, dans lequel lesdits émetteurs sont disposés sur ledit boîtier (20), de façon à affecter, grâce auxdites ondes, au cours dudit traitement, ladite partie du corps à traiter;
- dans lequel les moyens d'émission émettent des ondes électro-magnétiques sensiblement monochromatiques;
- dans lequel lesdits émetteurs (30) sont agencés de façon à géné-rer une matrice dans laquelle il est possible d'identifier des rangées et des colonnes;
- et dans lequel les émetteurs d'une rangée sont disposés dans une position intermédiaire par rapport aux émetteurs d'une rangée adjacente;
**caractérisé par le fait que** ledit corps de réception (2) présente une base de support (4), appropriée de façon à reposer sur ledit support, et une paroi supérieure (6), opposée à ladite base de support (4), dans lequel ladite paroi supérieure (6) présente un support (10) de manière à recevoir ledit boîtier (20).

2. Dispositif (1) selon la revendication 1, dans lequel lesdits émetteurs (30) sont appropriés de façon à émettre une onde qui présente une longueur d'onde sensiblement égale à la longueur d'onde caractéris-tique du silicium.

3. Dispositif (1) selon la revendication 2, dans lequel lesdits émetteurs (30) sont appropriés de façon à émettre une onde qui présente une longueur d'onde égale à 905 nanomètres.

4. Dispositif (1) selon l'une quelconque des revendications précéden-tes, dans lequel ledit boîtier de manipulation (20) présente une surface avant (22), prévue pour faire face à la partie à traiter au cours d'un traitement, et une surface arrière (24), lesdits émetteurs étant disposés sur le côté de ladite surface avant (22).

5. Dispositif (1) selon la revendication 4, dans lequel lesdits émetteurs (30) font saillie à partir de ladite surface avant (22).

6. Dispositif (1) selon l'une quelconque des revendications précéden-tes, dans lequel lesdits émetteurs (30) sont espacés les uns des autres.

7. Dispositif (1) selon l'une quelconque des revendications précéden-tes, dans lequel un émetteur (30) qui n'est pas disposé sur une rangée périphérique ou sur une colonne périphérique, présente une distance constante par rapport auxdits émetteurs adjacents (30).

8. Dispositif (1) selon l'une quelconque des revendications précéden-tes, dans lequel lesdits émetteurs (30) présentent un agencement structuré en nid d'abeilles.

9. Dispositif (1) selon l'une quelconque des revendications précéden-tes, dans lequel ledit support (10) présente un espace inférieur (12) et un espace supérieur (14), qui peuvent être distingués l'un de l'autre, ledit espace inférieur (12) étant approprié de façon à supporter lesdits émetteurs (30) et ledit espace supérieur (14) étant approprié de façon à supporter, au moins en partie, ledit boîtier (20).

10. Dispositif (1) selon l'une quelconque des revendications précéden-tes, comprenant des moyens d'alimentation secondaires appropriés de façon à alimenter lesdits émetteurs (30) de manière à émettre ladite onde.

11. Dispositif (1) selon la revendication 10, dans lequel lesdits moyens d'alimentation sont placés dans ledit boîtier (20).

12. Dispositif (1) selon l'une quelconque des revendications précéden-tes, comprenant des moyens d'alimentation primaires, appropriés de façon à coopérer avec lesdits moyens d'alimentation secondaires, de manière à alimenter lesdits moyens d'alimentation secondaires.

13. Dispositif (1) selon la revendication 12, dans lequel lesdits moyens d'alimentation primaires sont placés dans ledit corps de réception (2).

14. Dispositif (1) selon l'une quelconque des revendications 10 à 13, dans lequel lesdits moyens d'alimentation secondaires sont appropriés de façon à alimenter lesdits émetteurs en les alimentant à une fréquence prédéterminée.

15. Dispositif (1) selon la revendication 14, dans lequel lesdits moyens d'alimentation secondaires sont appropriés de façon à alimenter lesdits émetteurs en les alimentant à une fréquence égale à la fréquence du secteur.

16. Dispositif (1) selon l'une quelconque des revendications précéden-tes, dans lequel lesdits émetteurs (30) sont des diodes électro-luminescentes.

17. Assemblage destiné au traitement d'un corps humain ou animal, comprenant au moins un dispositif (1) selon l'une quelconque des revendications précédentes et au moins une paroi transparente vis-à-vis des ondes, de façon à empêcher un contact physique entre lesdits émetteurs (30) et ladite partie du corps à traiter.

18. Assemblage selon la revendication 17, dans lequel ladite paroi transparente constitue une partie d'un sac.

19. Assemblage selon la revendication 17 ou la revendication 18, dans lequel ladite paroi est réalisée en polyéthylène.
